# EUROPÄISCHE PATENTANMELDUNG

(11) **EP 0 625 340 A2**
(43) Veröffentlichungstag der Anmeldung: **23.11.1994**
(21) Anmeldenummer: 94103996.8
(22) Anmeldetag: 15.03.1994
(51) Int. Cl.: A61F 2/28

(54) **Manschette zur Heilbeschleunigung von Knochendefekten**

(30) Priorität: 22.04.1993 DE 4313192
(71) Anmelder: Kirsch, Axel, Dr., D-70794 Filderstadt (DE)
(72) Erfinder: Kirsch, Axel, Dr., D-70794 Filderstadt (DE)
(74) Vertreter: Goddar, Heinz J., Dr.

(57) **Zusammenfassung**

Vorrichtung zum vorübergehenden Abdecken einer mit Knochenaufbaumaterial (14), wie Hydroxylapatitgranulat, gefüllten Ausnehmung (12) in einem körpereigenen Knochen (10) mit einer Abdeckmembran (16) zwecks Heilbeschleunigung, wobei der Abdeckmembran eine Versteifungslage (17) zugeordnet ist, dadurch gekennzeichnet, daß eine biegsame Manschette (15), die die Abdeckmembran auf der dem Knochen zugewandten Seite und die Versteifungslage auf der dem Knochen abgewandten Seite umfaßt und zwei Verschlußkanten (15a,15b) aufweist, beim Umhüllen des Knochens, im Bereich der einen Knochendefekt bildenden Ausnehmung, mittels in die Versteifungslage eingreifender Verbindungselemente fest verschließbar ist.

## Beschreibung

Die Erfindung betrifft eine Vorrichtung zum vorübergehenden Abdecken einer mit Knochenaufbaumaterial, wie Hydroxylapatitgranulat, gefüllten Ausnehmung in einem körpereigenen Knochen mit einer Abdeckmembran zwecks Heilbeschleunigung, wobei der Abdeckmembran eine Versteifungslage zugeordnet ist.

Eine derartige Vorrichtung ist bereits in der (nicht vorveröffentlichten) deutschen Patentanmeldung P 43 02 709.1 beschrieben. Die dort beschriebene Abdeckeinrichtung zur Nachbehandlung von rekonstruierten Knochen in der plastischen Chirurgie oder von kieferchirurgischen Operationen gewährleistet, daß das Knochenaufbaumaterial im wesentlichen ausschließlich von der Knochenseite her knöchern durchwachsen wird, nicht aber in unerwünschter Weise von Schleimhautgewebe. So wird erreicht, daß sich die Knochendefektstelle im wesentlichen vollständig beseitigen und das Knochenaufbaumaterial nach knöchernem Durchwachsen in den körpereigenen Knochen integrieren läßt. Die Abdeckmembran, die Vorzugsweise aus resorbierbarem Material besteht, wie auch in der (nicht vorveröffentlichten) deutschen Patentanmeldung P 43 02 708.3 beschrieben, wird dabei zusammen mit der Versteifungslage allseits der Knochendefektstelle mittels Befestigungsnägeln, wie sie bspw. in der (ebenfalls nicht vorveröffentlichten) deutschen Patentanmeldung P 43 00 039.8 beschrieben sind, in dichter Anlage am körpereigenen Knochen befestigt. Zum einen bereitet das Einschlagen von Nägeln einem Patienten normalerweise Schmerz bzw. erfordert eine zumindest lokale Anästhesie, zum anderen müssen die Nägel nach dem Ausheilen der Knochendefektstelle und dem Abbau des Membranmaterials, wieder unter Zufügung von Schmerz bzw. unter lokaler Anästhesie, entfernt werden, um zu keinen Komplikationen Anlaß zu geben.

Der Erfindung liegt die Aufgabe zugrunde, eine Vorrichtung der gattungsgemäßen Art zu schaffen, die die obengenannten Nachteile überwindet, d. h. in den Knochen eingreifende Befestigungsmittel, wie bspw. Nägel, unnötig macht.

Erfindungsgemäß wird diese Aufgabe dadurch gelöst, daß eine biegsame Manschette, die die Abdeckmembran auf der dem Knochen zugewandten Seite und die Versteifungslage auf der dem Knochen abgewandten Seite umfaßt und zwei Verschlußkanten aufweist, beim Umhüllen des Knochens, im Bereich der einen Knochendefekt bildenden Ausnehmung, mittels in die Versteifungslage eingreifender Verbindungselemente fest verschließbar ist.

Dabei schlägt die Erfindung vor, daß die Versteifungslage senkrecht zu den Verschlußkanten höchstens die Länge des kleinsten Umfangs des abzudeckenden Knochens aufweist.

Erfindungsgemäß ist vorgesehen, daß die Abdeckmembran senkrecht zu den Verschlußkanten, von der einen Verschlußkante zu der anderen Verschlußkante, mindestens die Länge des größten Umfangs des abzudeckenden Knochens aufweist.

Die Erfindung schlägt auch vor, daß die Abdeckmembran senkrecht zu den Verschlußkanten, entlang der beiden Enden der Manschette, bündig mit der Versteifungslage abschließt.

Ferner ist erfindungsgemäß vorgesehen, daß die parallel zu den Verschlußkanten verlaufenden Kanten der Versteifungslage vom Knochen weggebogen sind.

Ein bevorzugtes Ausführungsbeispiel der Erfindung ist dadurch gekennzeichnet, daß die Abdeckmembran auf der Seite der einen Verschlußkante die Versteifungslage überragt und längs der anderen Verschlußkante dort endet, wo sich die Versteifungslage nach außen umbiegt.

Die Erfindung schlägt ferner vor, daß zumindest die Versteifungslage mindestens einen Schlitz senkrecht zu einer der Verschlußkanten aufweist, der sich von der entsprechenden, längs des Knochen verlaufenden Kante der Versteifungslage ausbreitet.

Dabei schlagt die Erfindung vor, daß die Manschette, Versteifungslage und Abdeckmembran, mindestens einen Schlitz senkrecht zu einer der Verschlußkanten aufweist, der sich von dieser Verschlußkante ausbreitet.

Erfindungsgemäß ist dabei auch vorgesehen, daß zwei durch einen Schlitz voneinander getrennnte Bereiche der Versteifungslage bündig aneinander anschließbar sind und sich zwei durch einen Schlitz voneinander getrennte Bereiche der Abdeckmembran überlappen.

Dabei kann vorgesehen sein, daß die Abdeckmembran längs einer Seite eines Schlitzes über die Versteifungslage herausragt und längs der anderen Seite bündig mit der Versteifungslage abschließt.

Ein bevorzugtes Ausführungsbeispiel der Erfindung ist ferner dadurch gekennzeichnet, daß pro Verschlußkante zwei Schlitze vorhanden sind, die im geschlossenen Zustand der Manschette im wesentlichen ineinander fluchten.

Erfindungsgemäß ist vorgeschlagen, daß sich ein Schlitz über maximal 1/5 des Umfangs des Knochens erstreckt.

Ferner schlägt die Erfindung vor, daß Befestigungselemente die Versteifungslage an der Abdeckmembran befestigen.

Ein bevorzugtes Ausfürungsbeispiel der Erfindung ist auch dadurch gekennzeichnet, daß die Befestigungselemente Nieten sind, die im wesentlichen miteinander fluchtende Durchführungen der Versteifungslage und der Abdeckmembran durchqueren.

Dabei ist erfindungsgemäß vorgesehen, daß die Nieten über die Abdeckmembran hinaus auf den Knochen ragen.

Dabei ist vorgesehen, daß im geschlossenen Zustand der Manschette die Verbindungselemente im wesentlichen miteinander fluchtende Durchführungen in den sich gegenüberliegenden Kanten der Versteifungslage durchqueren.

Die Erfindung schlägt dabei ferner vor, daß die umgebogenen Kanten der Versteifungslage von U-Form und die Durchführungen im wesentlichen längs der Verschlußkanten in der Mitte der U-Form der Versteifungslage angeordnet sind.

Dabei ist erfindungsgemäß vorgesehen, daß die Verbindungselemente durch einen schwer reißbaren Faden gebildet sind.

Die Erfindung schlägt alternativ dazu vor, daß die Verbindungselemente Stifte sind, deren Enden auf der der Abdeckmembran abgewandten Seite fest an der Versteifungslage anliegen, und mindestens ein Ende jedes Stiftes biegsam ist.

Erfindungsgemäß wird dabei vorgeschlagen, daß die Stifte jeweils ein biegsames Ende und ein Kopfende aufweisen.

Auch kann vorgesehen sein, daß die Stifte jeweils zwei biegsame Enden aufweisen.

Ferner ist erfindungsgemäß vorgesehen, daß die Manschette ein Scharnier umfaßt, das im wesentlichen parallel zu den Verschlußkanten angeordnet ist.

Dabei wird vorgeschlagen, daß das Scharnier im wesentlichen in der Mitte zwischen den umgebogenen Kanten der Versteifungslage angeordnet ist.

Nach der Erfindung kann schließlich auch vorgesehen sein, daß im geschlossenen Zustand der Manschette die Verschlußkanten benachbart zu der Ausnehmung auf dem Knochen aufliegen, so daß das Knochenaufbaumaterial dicht von der Abdeckmembran umgeben ist.

Der Erfindung liegt die überraschende Erkenntnis zugrunde, daß es gelingt, leicht und schmerzlos eine Knochendefektstelle mit einer Abdeckmembran fest zu umgeben, indem eine aus einer Abdeckmembran und einer Versteifungslage bestehende Manschette um die Knochendefektstelle gelegt wird, wobei der Umfang der Manschette beim Verbinden der Kanten der Versteifungslage an die jeweilige Knochenform durch Veränderung des Abstands zwischen diesen Kanten anpassbar ist. Der feste Halt der Manschette sorgt dafür, daß keine Bewegung der Abdeckmembran relativ zum Knochen stattfindet und das Blutkoagel ungestört in Knochengewebe überführt wird.

Weitere Merkmale und Vorteile der Erfindung ergeben sich aus der nachstehenden Beschreibung, in der Ausführungsbeispiele anhand der schematischen Zeichnung im einzelnen erläutert sind. Dabei zeigt:
- Fig. 1: einen Knochenbereich samt Knochendefektstelle mit einer unverschlossenen, erfindungsgemäßen Manschette, schräg von vorne;
- Fig. 2: einen Schnitt durch die in Fig. 1 gezeigte Manschette im Bereich einer Befestigungsniete;
- Fig. 3: eine Manschette gemäß einem Ausführungsbeispiel der Erfindung, schräg von vorne;
- Fig. 4: eine Manschette gemäß einem anderen Ausführungsbeispiel der Erfindung, schräg von vorne;
- Fig. 5a: eine Draufsicht auf den in Fig. 1 gezeigten Knochenbereich mit über der Knochendefektstelle verschlossener Manschette; und
- Fig. 5b: eine Teilvergrößerung des in Fig. 5a gezeigten Manschettenbundbereichs I mit einem Verbindungsstift.

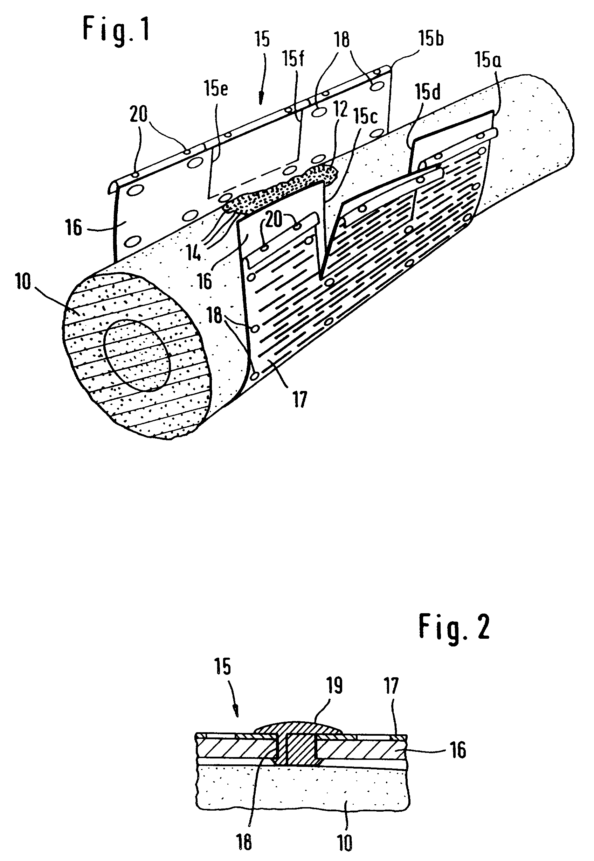

In Fig. 1 ist ein körpereigener Knochen 10 mit einer eine Knochendefektstelle bildenden Ausnehmung 12 dargestellt, wobei die Ausnehmung 12 mit Knochenaufbaumaterial 14, wie Hydroxylapatitgranulat vermengt mit aus körpereigenem Knochengewebe bestehenden Knochenpartikeln, im wesentlichen vollständig gefüllt ist. Der Knochen 10 ist, außer im Bereich der Knochendefektstelle 12, mit einer Manschette 15 umgeben, deren noch nicht miteinander verbundenen, parallel zur Knochenachse verlaufenden Verschlußkanten 15a, 15b benachbart zur Knochendefektstelle 12 vom Knochen 10 abstehen.

Eine Abdeckmembran 16 ist auf der dem Knochen 10 zugewandten Seite und eine Versteifungslage 17 auf der dem Knochen 10 abgewandten Seite der Manschette 15 angeordnet.

Die sich gegenüberliegenden, parallel zu den Verschlußkanten 15a, 15b verlaufenden Kanten der Versteifungslage 17 sind jeweils von der Abdeckmembran 16 weggebogen und weisen eine U-Form auf.

Senkrecht zu den Verschlußkonten 15a, 15b schließt die Abdeckmembran 16 entlang der beiden Enden der Manschette 15 bündig mit der Versteifungslage 17 ab. Auf der Seite der einen Verschlußkante 15a der Manschette 15 überragt die Abdeckmembran 16 die Verschlußkante 15a, und längs der anderen Verschlußkante 15b endet die Abdeckinembran 16 dort, wo sich die Kanten der Versteifungslage 17 nach außen wegbiegen. Hierbei weist die Abdeckmembran 16 von der einen Verschlußkante 15a zu der anderen Verschlußkante 15b mindestens die Länge des maximalen Umfangs des abzudeckenden Knochens 10 auf, was ein dichtes Umschließen der Manschette 15 ermöglicht.

Senkrecht zu den beiden Verschlußkanten 15a, 15b der Manschette 15 sind jeweils zwei Schlitze 15c, 15d, 15e, 15e angeordnet, wobei sich das eine Paar von Schlitzen 15c, 15d von der einen Verschlußkante 15a über ungefähr 1/5 des Umfangs der Manschette 15 in Richtung der anderen Verschlußkante 15b und das andere Paar von Schlitzen 15e, 15f von der anderen Verschlußkante 15b über ungefähr 1/5 des Umfangs der Manschette 15 in Richtung der einen Verschlußkante 15a erstreckt. Hierdurch werden pro Verschlußkante 15a, 15b drei Manschettenbereiche definiert.

Während die drei durch die Schlitze 15c, 15d bzw. 15e, 15f voneinander getrennten Bereiche der Versteifungslage 17 beim Schließen der Schlitze 15c bzw. 15d, 15e, 15f bündig aneinander anliegen, überlappen sich die entsprechenden Bereiche der Abdeckmembran 16, da die Abdeckmembran 16 und die Versteifungslage 17 nur im Manschettenbereich zwischen den Schlitzen 15c, 15d bzw. 15e, 15f längs dieser Schlitze 15c, 15d bzw. 15e, 15f bündig miteinander abschließen, und bei den beiden anderen Manschettenbereichen die Abdeckmembran 16 entlang der Schlitze 15c, 15d bzw. 15e, 15f die Versteifungslage 17 überragt, so daß ein dichtes Schließen der Schlitze 15c, 15d, 15e, 15f möglich ist.

Wie Fig. 2 entnommen werden kann, ist die Versteifungslage 17 mittels Befestigungsnieten 19 an der Abdeckmembran 16 befestigt. Hierbei greifen die Befestigungsnieten 19 durch in der Abdeckmembran 16 und der Versteifungslage 17 vorhandene, ineinander fluchtende Durchführungen 18 und überragen die Abdeckmembran 16, so daß sie im geschlossenen Zustand der Manschette 15 auf den Knochen 10 drücken und somit den Halt der Manschette 15 auf dem Knochen 10 festigen und die Reibung zwischen der Abdeckmembran 16 und dem Knochenaufbaumaterial 14 minimieren.

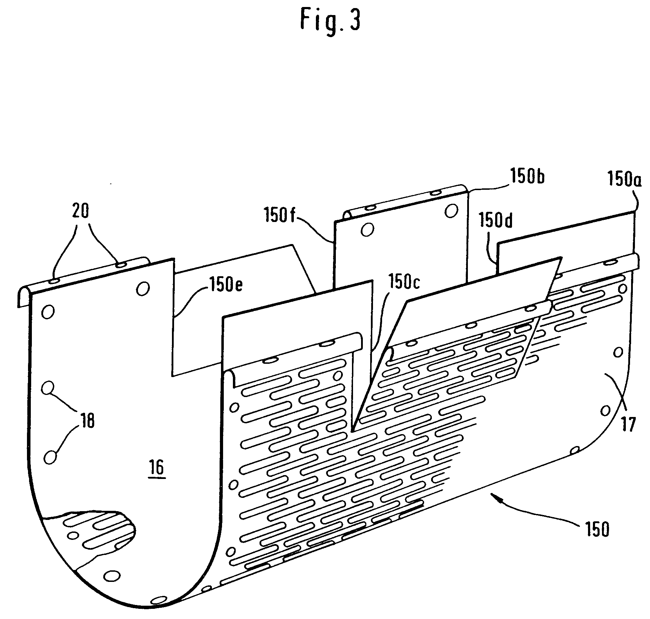

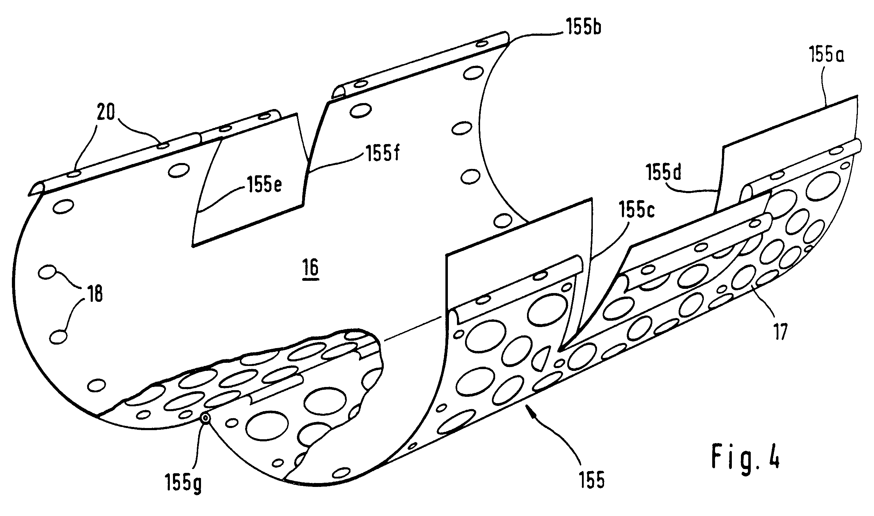

Zum Abdecken der Knochendefektstelle 12 wird die in Fig. 3 dargestellte Manschette 150 von der der Knochendefektstelle 12 gegenüberliegenden Seite des Knochens 10 über den Knochen 10 geschoben, während die in Fig. 4 dargestellte Manschette 155 um den Knochen 10 herumgelegt werden kann, da sie mittig zwischen und parallel zu den umgebogenen Kanten ihrer Versteifungslage 17 ein Scharnier 155g aufweist.

Wie in Fig. 1 für die Manschette 15 dargestellt, stehen die Verschlußkanten 150a, 150b bzw. 155a, 155b der nicht verschlossenen Manschetten 150 bzw. 155 dann benachbart zur Knochendefektstelle 12 mit ihren durch die Schlitze 150c, 150d, 150e, 150f bzw. 155c, 155d, 155e, 155f gekennzeichneten Manschettenbereiche vom Knochen 10 ab.

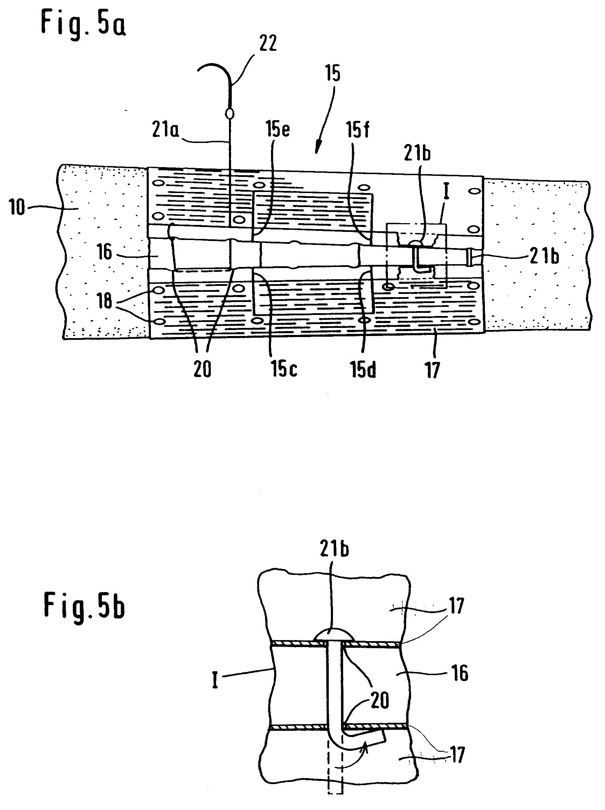

Die in Fig. 1 gezeigte, nicht verschlossene Manschette 15 wird wie folgt in den in Fig. 5a und 5b dargestellten, geschlossenen Zustand gebracht:
Zuerst wird die eine Verschlußkante 15a, die nur die Abdeckmembran 16 aufweist, auf den Knochen 10 gedrückt, so daß die Knochendefektstelle 12 mit der Abdeckmembran 16, abgedeckt ist, wobei keine Befestigungsnieten 19 auf dem Knochenaufbaumaterial 14 aufliegen.

Dann wird auch die andere Verschlußkante 15b der Manschette 15 auf den Knochen 10 gedückt, so daß die Knochendefektstelle 12 mit mindestens einer Lage Abdeckmaterial 16 abgedeckt ist, wobei sich die umgebogenen Kanten der Versteifungslage 17 nicht berühren, wie in Fig. 5a und 5b zu sehen ist, da die Versteifungslage 17 von ihrer einen umgebogenen Kante zu ihrer anderen umgebogenen Kante höchstens die Länge des minimalen Umfangs des zu bedeckenden Knochens 10 aufweist.

Als nächstes werden die umgebogenen Kanten der Versteifungslage 17 so nah wie möglich aneinader gebracht, so daß in Anpassung an die Gestalt des Knochens 10 die einen drei durch die Schlitze 15c, 15d gebildeten Bereiche mit unterschiedlichen Abständen den anderen drei durch die Schlitze 15e, 15f gebildeten Bereiche der Versteifungslage 17 gegenüberstehen. Bei dem in Fig. 5a gezeigten, sich nach rechts verjüngenden Knochen 10 kommen sich die am rechten Rand der Manschette 15 liegenden Bereiche der Versteifungslage 17 am nächsten und die am linken Rand liegenden Bereiche sind am weitesten voneinander entfernt. Die Schlitze 15c, 15d, 15e, 15f unterdrücken im westlichen das Auftreten von Falten auf Grund des sich mit dem Knochenumfang verändernden Abstands zwischen den sich gegenüberliegenden, umgebogenen Kanten der Versteifungslage 17.

Schließlich werden die umgebogenen Kanten der Versteifungslage 17, die mit Durchführungen 20 versehen sind, wie deutlich in den Fig.en 1, 3 und 4 zu sehen, fest miteinander verbunden, indem Verbindungselemente 21a, 21b durch sich im wesentlichen gegenüberliegende, miteinander fluchtende Durchführungen 20 gesteckt werden, siehe Fig. 5a und 5b.

Mit einem an einem Haken 22 befestigten, schwer reißbaren Faden 21a kann die Manschette 15 zugenäht werden, indem der Haken 22 jeweils durch zwei sich gegenüberliegende Durchführungen 20 geschoben wird. Form und Halt der Manschette 15 hängen hierbei von der Spannung des Fadens 21a ab.

Ein Verbindungsstift 21b, der an seinem einen Ende einen Kopf aufweist, kann mit seinem anderen, geraden Ende durch zwei im wesentlichen ineinander fluchtende Durchführungen 20 in zwei sich gegenüberliegenden Kanten der Versteifungslage 17 gesteckt werden, bis der Kopf auf der Versteifungslage 17 zum Aufliegen kommt. Das gerade Ende des Verbindungsstiftes 21b, muß dann umgebogen werden, um die Manschette 15 fest zu verschließen, wie in Fig. 5b dargestellt. Form und Halt der Manschette 15 hängen hierbei davon ab, an welchen Stellen die die Durchführungen 20 durchquerenden Verbindungsstifte 21b umgebogen werden.

Alle in ihrer Länge einstellbaren, fest mit den Kanten der Versteifungslage verbindbaren Verbindungselemente können zum Verschließen einer erfindungsgemäßen Manschette 15, 150, 155 verwendet werden.

Die Versteifungslage 17 kann mit Hilfe irgendeines der bekannten Befestigungsmittel an die Abdeckmembran 16 angebracht werden, wobei dieses Befestigungsmittel vorteilhafterweise an mehreren Stellen die Abdeckmembran 16 überragen sollte, um gleichzeitig auf den Knochen 10 zu drücken und den Halt einer Manschette 15, 150, 155 gemäß der Erfindung zu sichern.

Die Abdeckmembran 16 kann identisch mit der in der (nicht vorveröffentlichten) deutschen Patentanmeldung P 43 02 708.3 und die Versteifungslage kann identisch mit der in der (nicht vorveröffentlichten) deutschen Patentanmeldung P 43 02 709.1 offenbarten sein.

Knochen, die ähnliche Dimensionen haben, aber nicht identisch sein müssen, können jeweils von einer standardisierten Manschette 15, 150, 155 gemäß der Erfindung fest umhüllt werden, da der Abstand zwischen den umgebogenen Kanten der Versteifungslage 17 anpaßbar an die jeweilige Knochenform ist, während die Dimensionierung der Abdeckmembran 16 sicherstellt, daß die jeweilige Knochendefektstelle 12 von Membranmaterial abgedeckt wird.

Sobald das knöcherne Durchwachsen des Knochenaufbaumaterials 14 im wesentlichen abgeschlossen und, im Falle einer Abdeckmembran 16 aus resorbierbarem Material, die Abdeckmembran 16 im wesentlichen abgebaut ist, kann die Manschette 15, 150, 155 durch Entfernen der Verbindungselemente 21a, 21b vom Knochen 10 abgenommen werden.

Die in der vorstehenden Beschreibung, in der Zeichnung sowie in den Ansprüchen offenbarten Merkmale der Erfindung können sowohl einzeln als auch in beliebiger Kombination für die Verwirklichung der Erfindung in ihren verschiedensten Ausführungsformen wesentlich sein.

### BEZUGSZEICHENLISTE

- 10: Knochen
- 12: Ausnehmung
- 14: Knochenaufbaumaterial
- 15: Manschette
- 15a: Verschlußkante
- 15b: Verschlußkante
- 15c: Schlitz
- 15d: Schlitz
- 15e: Schlitz
- 15f: Schlitz
- 16: Abdeckmembran
- 17: Versteifungslage
- 18: Durchführung
- 19: Befestigungselement
- 20: Durchführung
- 21a: Faden
- 21b: Verbindungsstift
- 22: Nadel
- 150: Manschette
- 150a: Verschlußkante
- 150b: Verschlußkante
- 150c: Schlitz
- 150d: Schlitz
- 150e: Schlitz
- 150f: Schlitz
- 155: Scharnier
- 155: Manschette
- 155a: Verschlußkante
- 155b: Verschlußkante
- 155c: Schlitz
- 155d: Schlitz
- 155e: Schlitz
- 155f: Schlitz
- 155g: Scharnier

## Patentansprüche

1. Vorrichtung zum vorübergehenden Abdecken einer mit Knochenaufbaumaterial, wie Hydroxylapatitgranulat, gefüllten Ausnehmung in einem körpereigenen Knochen mit einer Abdeckmembran zwecks Heilbeschleunigung, wobei der Abdeckmembran eine Versteifungslage zugeordnet ist,
dadurch gekennzeichnet, daß
eine biegsame Manschette (15, 150, 155), die die Abdeckmembran (16) auf der dem Knochen (10) zugewandten Seite und die Versteifungslage (17) auf der dem Knochen (10) abgewandten Seite umfaßt und zwei Verschlußkanten (15a, 15b, 150a, 150b, 155a, 155b) aufweist, beim Umhüllen des Knochens (10), im Bereich der einen Knochendefekt bildenden Ausnehmung (12), mittels in die Versteifungslage (17) eingreifender Verbindungselemente (21a, 21b) fest verschließbar ist.

2. Vorrichtung nach Anspruch 1,
dadurch gekennzeichnet, daß
die Versteifungslage (17) senkrecht zu den Verschlußkanten (15a, 15b, 150a, 150b, 155a, 155b) höchstens die Länge des kleinsten Umfangs des abzudeckenden Knochens (10) aufweist.

3. Vorrichtung nach Anspruch 1 oder 2,
dadurch gekennzeichnet, daß
die Abdeckmembran (16) senkrecht zu den Verschlußkanten (15a, 15b, 150a, 150b, 155a, 155b), von der einen Verschlußkante (15a, 150a, 155a) zu der anderen Verschlußkante (15b, 150b, 155b), mindestens die Länge des größten Umfangs des abzudeckenden Knochens (10) aufweist.

4. Vorrichtung nach einem der Ansprüche 1 bis 3,
dadurch gekennzeichnet, daß
die Abdeckmembran (16) senkrecht zu den Verschlußkanten (15a, 15b, 150a, 150b, 155a, 155b), entlang der beiden Enden der Manschette (15, 150, 155), bündig mit der Versteifungslage (17) abschließt.

5. Vorrichtung nach einem der Ansprüche 1 bis 4,
dadurch gekennzeichnet, daß
die parallel zu den Verschlußkanten (15a, 15b, 150a, 150b, 155a, 155b) verlaufenden Kanten der Versteifungslage (17) vom Knochen (10) weggebogen sind.

6. Vorrichtung nach Anspruch 5,
dadurch gekennzeichnet, daß
die Abdeckmembran (16) auf der Seite der einen Verschlußkante (15a, 150a, 155a) die Versteifungslage (17) überragt und längs der anderen Verschlußkante (15b, 150b, 155b) dort endet, wo sich die Versteifungslage (17) nach außen umbiegt.

7. Vorrichtung nach einem der Ansprüche 1 bis 6,
dadurch gekennzeichnet, daß
zumindest die Versteifungslage (17) mindestens einen Schlitz (15c, 15d, 15e, 15f, 150c, 150d, 150e, 150f, 155c, 155d, 155e, 155f) senkrecht zu einer der Verschlußkanten (15a, 15b, 150a, 150b, 155a, 155b) aufweist, der sich von der entsprechenden, längs des Knochens (10) verlaufenden Kante der Versteifungslage (17) ausbreitet.

8. Vorrichtung nach Anspruch 7,
dadurch gekennzeichnet, daß
die Manschette (15, 150, 155), Versteifungslage (17) und Abdeckmembran (16), mindestens einen Schlitz (15c, 15d, 15e, 15f, 150c, 150d, 150e, 150f, 155c, 155d, 155e, 155f) senkrecht zu einer der Verschlußkanten (15a, 15b, 150a, 150b, 155a, 155b) aufweist, der sich von dieser Verschlußkante ausbreitet.

9. Vorrichtung nach Anspruch 8,
dadurch gekennzeichnet, daß
zwei durch einen Schlitz (15c, 15d, 15e, 15f, 150c, 150d, 150e, 150f, 155c, 155d, 155e, 155f) voneinander getrennnte Bereiche der Versteifungslage (17) bündig aneinander anschließbar sind und sich zwei durch einen Schlitz (15c, 15d, 15e, 15f, 150c, 150d, 150e, 150f, 155c, 155d, 155e, 155f) voneinander getrennte Bereiche der Abdeckmembran (16) überlappen.

10. Vorrichtung nach Anspruch 9,
dadurch gekennzeichnet, daß
die Abdeckmembran (16) längs einer Seite eines Schlitzes (15c, 15d, 15e, 15f, 150c, 150d, 150e, 150f, 155c, 155d, 155e, 155f) über die Versteifungslage (17) herausragt und längs der anderen Seite bündig mit der Versteifungslage (17) abschließt.

11. Vorrichtung nach einem der Ansprüche 7 bis 10,
dadurch gekennzeichnet, daß
pro Verschlußkante (15a, 15b, 150a, 150b, 155a, 155b) zwei Schlitze (15c, 15d, 15e, 15f, 150c, 150d, 150e, 150f, 155c, 155d, 155e, 155f) vorhanden sind, die im geschlossenen Zustand der Manschette (15, 150, 155) im wesentlichen ineinander fluchten.

12. Vorrichtung nach einem der Ansprüche 7 bis 11,
dadurch gekennzeichnet, daß
sich ein Schlitz (15c, 15d, 15e, 15f, 150c, 150d, 150e, 150f, 155c, 155d, 155e, 155f) über maximal 1/5 des Umfangs des Knochens (10) erstreckt.

13. Vorrichtung nach einem der Ansprüche 1 bis 12,
dadurch gekennzeichnet, daß
Befestigungselemente (19) die Versteifungslage (17) an der Abdeckmembran (16) befestigen.

14. Vorrichtung nach Anspruch 13,
dadurch gekennzeichnet, daß
die Befestigungselemente Nieten (19) sind, die im wesentlichen miteinander fluchtende Durchführungen (18) der Versteifungslage (17) und der Abdeckmembran (16) durchqueren.

15. Vorrichtung nach Anspruch 14,
dadurch gekennzeichnet, daß
die Nieten (19) über die Abdeckmembran (16) hinaus auf den Knochen (10) ragen.

16. Vorrichtung nach einem der Ansprüche 1 bis 15,
dadurch gekennzeichnet, daß
im geschlossenen Zustand der Manschette (15, 150, 155) die Verbindungselemente (21a, 21b) im wesentlichen miteinander fluchtende Durchführungen (20) in den sich gegenüberliegenden, Kanten der Versteifungslage (17) durchqueren.

17. Vorrichtung nach Anspruch 16,
dadurch gekennzeichnet, daß
die umgebogenen Kanten der Versteifungslage (17) von U-Form und die Durchführungen (20) im wesentlichen längs der Verschlußkanten (15a, 15b, 150a, 150b, 155a, 155b) in der Mitte der U-Form der Versteifungslage (17) angeordnet sind.

18. Vorrichtung nach Anspruch 16 oder 17,
dadurch gekennzeichnet, daß
die Verbindungselemente durch einen schwer reißbaren Faden (21a) gebildet sind.

19. Vorrichtung nach Anspruch 16 oder 17,
dadurch gekennzeichnet, daß
die Verbindungselemente Stifte (21b) sind, deren Enden auf der der Abdeckmembran (16) abgewandten Seite fest an der Versteifungslage (17) anliegen, und mindestens ein Ende jedes Stiftes (21b) biegsam ist.

20. Vorrichtung nach Anspruch 19,
dadurch gekennzeichnet, daß
die Stifte (21b) jeweils ein biegsames Ende und ein Kopfende aufweisen.

21. Vorrichtung nach Anspruch 19,
dadurch gekennzeichnet, daß
die Stifte jeweils zwei biegsame Enden aufweisen.

22. Vorrichtung nach einem der Ansprüche 1 bis 21,
dadurch gekennzeichnet, daß
die Manschette (155) ein Scharnier (155g) umfaßt, das im wesentlichen parallel zu den Verschlußkanten (155a, 155b) angeordnet ist.

23. Vorrichtung nach Anspruch 22,
dadurch gekennzeichnet, daß
das Scharnier (155g) im wesentlichen in der Mitte zwischen den umgebogenen Kanten der Versteifungslage (17) angeordnet ist.

24. Vorrichtung nach einem der Ansprüche 1 bis 23,
dadurch gekennzeichnet, daß
im geschlossenen Zustand der Manschette (15, 150, 155) die Verschlußkanten (15a, 15b, 150a, 150b, 155a, 155b) benachbart zu der Ausnehmung (12) auf dem Knochen (10) aufliegen, so daß das Knochenaufbaumaterial (14) dicht von der Abdeckmembran (16) umgeben ist.
